# EUROPEAN PATENT APPLICATION

(11) **EP 4 170 355 A1**
(43) Date of publication of application: **26.04.2023**
(21) Application number: 21829605.1
(22) Date of filing: 22.06.2021
(51) Int. Cl.: G01P 13/00, A61B 3/16, A61B 5/01

(54) **METHOD FOR ESTIMATING FLUID STATE OF LIQUID, AND SYSTEM FOR ESTIMATING FLUID STATE OF LIQUID**

(30) Priority: 23.06.2020 JP 2020108282
(71) Applicant: KOWA COMPANY, LTD., Naka-ku Nagoya-shi Aichi 460-8625 (JP)
(72) Inventor: SUGIURA Yuka, Higashimurayama-shi, Tokyo 189-0022 (JP); YABUSAKI Katsumi, Higashimurayama-shi, Tokyo 189-0022 (JP); SHINOHARA Takao, Higashimurayama-shi, Tokyo 189-0022 (JP)
(74) Representative: LKGLOBAL Lorenz & Kopf PartG mbB Patentanwälte
(86) International application number: PCT/JP2021/023542
(87) International publication number: WO 2021/261472

(57) **Abstract**

The method for estimating the fluid state of a liquid of the present invention includes a step (a) of applying heat to the liquid, a step (b) of acquiring a temperature distribution of the liquid to which the heat is applied, and a step (c) of estimating the fluid state of the liquid based on the acquired temperature distribution. The system for estimating the fluid state of a liquid of the present invention includes a heating means (1) that applies heat to the liquid, a temperature distribution acquisition means (2) that acquires a temperature distribution of the liquid to which the heat is applied, and an estimation means (3) that estimates the fluid state of the liquid based on the acquired temperature distribution. According to such method and system for estimating the fluid state of a liquid, the flow of the liquid can be estimated non-invasively.

## Description

### [Technical Field]

The present invention relates to a method and a system for estimating the fluid state of a liquid.

### [Background Art]

Aqueous humor has roles to supply nutrients to intraocular avascular tissues, remove waste products, and regulate intraocular pressure. Normally, the intraocular pressure is kept constant by circulation in which aqueous humor produced in the eye passes through trabecula and is drained out of the eye through the Schlemm's canal. However, if the trabecula becomes clogged, the fluidity and drainage function of the aqueous humor will be lowered to increase the intraocular pressure. Increased intraocular pressure is known as one of the dominant mechanisms of glaucoma onset, and the optic nerve is compressed, which may result in lowered visual function and symptoms such as narrowed visual field and lowered visual acuity. Therefore, perceiving the flow of aqueous humor in the eye is important in early detection and early treatment of glaucoma.

As a technique for perceiving the flow of aqueous humor in the eye, for example, Patent Document 1 discloses locally administering a traceable component such as a dye or a fluorescent substance to the aqueous humor to visualize and monitor the flow of the aqueous humor.

### [Prior Art Documents]

### [Patent Documents]

[Patent Document 1] WO2003/073968

### [Summary of the Invention]

### [Problems to be solved by the Invention]

In the technique disclosed in Patent Document 1, however, it is necessary to use a syringe or the like in order to administer the traceable component to the aqueous humor, and in consideration of the administration to the aqueous humor in the eye, the above technique may be undesired from the viewpoint of invasiveness.

In addition to perceiving the flow of aqueous humor in the eye, there are needs in various fields to non-invasively perceive the extremely weak flow of a liquid (micro flow rate) and the flow of an invisible liquid. For example, such specific needs include perceiving the flow state of water or oil in order to find the presence or absence of water or oil leaks and the site of their causes, perceiving the flow state of blood in the human body, verification of the processing accuracy of microchannels, etc.

The present invention has been made in view of the above problems, and objects of the present invention include providing a method for estimating the fluid state of a liquid and a system for estimating the fluid state of a liquid that are able to non-invasively estimate the fluid state of a liquid.

### [Means for solving the Problems]

To achieve the above objects, first, the present invention provides a method for estimating the fluid state of a liquid, including: a step (a) of applying heat to the liquid; a step (b) of acquiring a temperature distribution of the liquid to which the heat is applied; and a step (c) of estimating the fluid state of the liquid based on the acquired temperature distribution (Invention 1).

According to the above invention (Invention 1), the fluid state of the liquid is estimated by perceiving the temperature distribution of the liquid, to which heat is applied, while taking advantage of the property that heat can be applied non-invasively to the liquid, and the fluid state of the liquid can therefore be estimated non-invasively.

In the above invention (invention 1), the step (b) may include acquiring a plurality of temperature distributions of the liquid at different time points, and the step (c) may include estimating the fluid state of the liquid based on the acquired plurality of temperature distributions (Invention 2).

According to the above invention (Invention 2), the fluid state of the liquid is comprehensively estimated based on the plurality of temperature distributions, and the estimation accuracy can thereby be improved as compared with the case in which the fluid state of the liquid is estimated from one temperature distribution.

In the above invention (Invention 1, 2), the step (c) may include: a step (c1) of setting two positions to obtain a temperature difference or temperature ratio between the two positions, the two positions forming a pair within a region in which the temperature distribution is acquired; a step (c2) of executing the step (c1) a plurality of times to select one pair from among a plurality of pairs of the two points within the region in which the temperature distribution is acquired, the one pair having a maximum temperature difference or temperature ratio; and a step (c3) of estimating, as a direction in which the liquid flows, a direction from a position on a lower temperature side to a position on a higher temperature side of the two positions constituting the selected one pair (Invention 3).

In the above invention (Invention 3), the step (c) may further include a step (c4) of estimating a rate at which the liquid flows from the temperature difference or temperature ratio between the two positions constituting the one pair selected in the step (c2) (Invention 4).

In the above invention (Invention 1 to 4), the step (c) may include dividing a region in which the temperature distribution is acquired into a plurality of unit regions and estimating the fluid state of the liquid based on temperatures of the plurality of unit regions (Invention 5).

In the above invention (Invention 5), the temperature of one of the unit regions may be calculated as an average value of the temperature of the one of the unit regions determined by the temperature distribution and the temperature of another unit region adjacent to the one of the unit regions (Invention 6).

In the above invention (Invention 1 to 6), the step (b) may include acquiring at least one of the temperature distribution of the liquid during a heating period in which the heat is applied to the liquid and the temperature distribution of the liquid during a cooling period after application of the heat to the liquid is stopped, and the step (c) may include estimating the fluid state of the liquid based on at least one of the temperature distribution during the heating period and the temperature distribution during the cooling period (Invention 7).

In the above invention (Invention 1 to 7), the step (a) may include irradiating the liquid with light from a light source thereby to apply the heat to the liquid (Invention 8).

In the above invention (Invention 1 to 8), the step (b) may include capturing an image of the liquid with a thermography camera thereby to acquire the temperature distribution of the liquid (Invention 9).

Second, the present invention provides a system for estimating the fluid state of a liquid, including: a heating means that applies heat to the liquid; a temperature distribution acquisition means that acquires a temperature distribution of the liquid to which the heat is applied; and an estimation means that estimates the fluid state of the liquid based on the acquired temperature distribution (Invention 10).

### [Advantageous Effect of the Invention]

According to the method for estimating the fluid state of a liquid and the system for estimating the fluid state of a liquid of the present invention, the fluid state of the liquid is estimated by perceiving the temperature distribution of the liquid, to which heat is applied, while taking advantage of the property that heat can be applied non-invasively to the liquid, and the fluid state of the liquid can therefore be estimated non-invasively.

### [Brief Description of Drawings]

FIG. 1 is a schematic configuration diagram of a system for estimating the fluid state of a liquid according to an embodiment of the present invention.
FIG. 2 is a block diagram illustrating the configuration of an estimation device of the system for estimating the fluid state of a liquid according to the embodiment.
FIG. 3 is a functional block diagram for describing functions that play major roles in the estimation device of the system for estimating the fluid state of a liquid according to the embodiment.
FIG. 4 is an explanatory diagram schematically illustrating a temperature distribution image of a liquid to which heat is applied by the system for estimating the fluid state of a liquid according to the embodiment.
FIG. 5 is an explanatory diagram schematically illustrating a state in which an analysis region is set for the temperature distribution image of a liquid.
FIG. 6 is a set of explanatory diagrams schematically illustrating how to estimate a direction in which the liquid flows in the analysis region of the temperature distribution image.
FIG. 7 is a flowchart illustrating a flow of estimating the fluid state of a liquid by the system for estimating the fluid state of a liquid according to the embodiment.
FIG. 8 is a set of explanatory diagrams schematically illustrating modified examples of analysis regions, in which FIG. 8(a) illustrates an example of setting an analysis region composed of square unit regions while FIG. 8(b) illustrates an example of setting a circular analysis region by dividing concentric circles into a plurality of unit regions so that the area of the unit regions is the same.
FIG. 9 is a set of schematic configuration diagrams of an evaluation device used in examples.
FIG. 10 is a set of diagrams schematically illustrating temperature distribution images of Example 1 representing temporal changes in the temperature distribution.
FIG. 11 is a set of diagrams schematically illustrating temperature distribution images of Example 2 representing differences in the temperature distribution due to differences in the flow rate.
FIG. 12 is a set of diagrams each illustrating a state in which two regions are set in the temperature distribution image obtained from an example.
FIG. 13 is a graph illustrating temporal changes in the temperature difference between two regions.
FIG. 14 is a graph illustrating the amount of change in the temperature difference for each flow rate pattern.

### [Embodiments for Carrying out the Invention]

Hereinafter, one or more embodiments of the present invention will be described with reference to the drawings. The embodiments described below are exemplified, and the present invention is not limited to these embodiments.

The present embodiment will be described with reference to an exemplary case in which the fluid state of aqueous humor of a subject's eye E is estimated by a system 100 for estimating the fluid state of a liquid. The fluid state of aqueous humor refers to a concept that encompasses the presence or absence of an aqueous humor flow and the direction and flow rate of the aqueous humor flow. In the present embodiment, the system 100 is used to confirm the presence or absence of an aqueous humor flow and estimate the direction and flow rate of the aqueous humor flow.

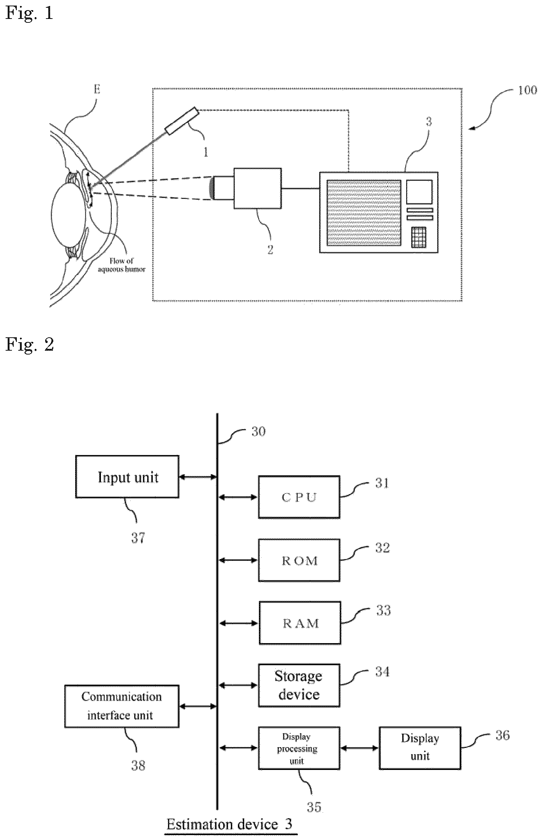

As illustrated in FIG. 1, the system 100 according to the present embodiment includes a light source 1 that irradiates the subject's eye E with near-infrared light to apply heat to the aqueous humor of the subject's eye E, a thermography camera 2 that captures one or more images of the subject's eye E in order to acquire the temperature distribution of the aqueous humor of the subject's eye E to which heat is applied from the light source 1, and an estimation device 3 that estimates the fluid state of the aqueous humor from the acquired temperature distribution information (captured temperature distribution image or images) of the aqueous humor.

As the light source 1 used for applying heat to the aqueous humor of the subject's eye E in the present embodiment, for example, a halogen lamp (halogen heater) capable of emitting near-infrared light can be used. By continuing to irradiate the subject's eye E with near-infrared light from the light source 1 for a predetermined time period, for example, 20 seconds, the near-infrared light passes through the cornea of the subject's eye E to enter the aqueous humor and can heat the aqueous humor. The light source 1 is an example of the "heating means" in the present invention and is not limited to this, provided that it can apply heat to a liquid. For example, a laser light source, an LED light source, or the like may be used. As substitute for the light source 1, a fomentation device, a hot towel, or the like may also be adopted as the "heating means."

The position at which the subject's eye E is irradiated with near-infrared light from the light source 1 is perceived as the heating position of the subject's eye E, but in order to facilitate the perception of the heating position, the subject's eye E may be irradiated with light beams at different angles from the light source 1 including two or more light sources, and a site at which the two or more light beams intersect may be perceived as the heating position.

The thermography camera 2 is a device that analyzes infrared rays radiated from the aqueous humor of the subject's eye E, to which heat is applied, and captures one or more images of the temperature distribution in a predetermined region, and a known infrared thermography camera can be used. The thermography camera 2 is connected to the estimation device 3 and is configured to be able to transmit the acquired temperature distribution information (captured temperature distribution image data) of the aqueous humor to the estimation device 3. The thermography camera 2 is an example of the "temperature distribution acquisition means" in the present invention.

The thermography camera 2 captures one or more images of the subject's eye E from the time point at which the light source 1 starts irradiation of the subject's eye E with near-infrared light from the light source 1, and acquires the temperature distribution information of the subject's eye E (its aqueous humor). For example, the thermography camera 2 acquires the temperature distribution information of the subject's eye E at image capture intervals of 1 frame per second for 20 seconds from the time point at which the light source 1 starts the irradiation. The acquired temperature distribution information of the subject's eye E is transmitted from the thermography camera 2 to the estimation device 3 as the temperature distribution images of the subject's eye E. The temperature distribution images transmitted from the thermography camera 2 to the estimation device 3 can be displayed on a display unit 36 of the estimation device 3, which will be described later.

The estimation device 3 is configured to acquire the temperature distribution information transmitted from the thermography camera 2 and estimate the fluid state of the aqueous humor based on the acquired temperature distribution information and can visualize the estimated fluid state of the aqueous humor. The estimation device 3 is an example of the "estimation means" in the present invention. For example, a general-purpose personal computer can be used as the estimation device 3.

As illustrated in FIG. 2, the estimation device 3 includes a CPU 31, a ROM 32, a RAM 33, a storage device 34, a display processing unit 35, a display unit 36, an input unit 37, and a communication interface unit 38, and a bus 30 is provided for transmitting control signals or data signals between the units.

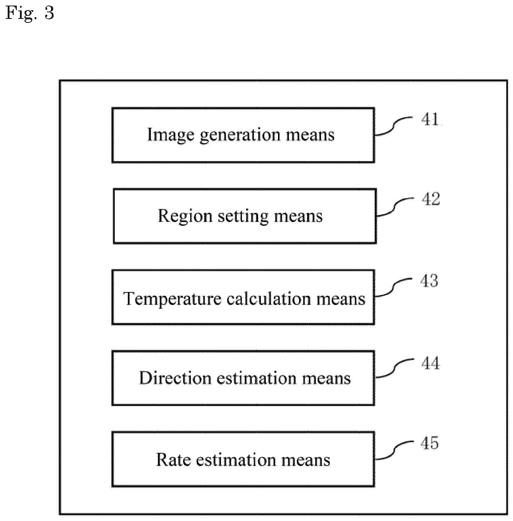

When the estimation device 3 is powered on, the CPU 31 loads various programs stored in the ROM 32 or storage device 34 into the RAM 33 and executes the programs. In the present embodiment, the CPU 31 reads out and executes the programs stored in the ROM 32 or storage device 34 thereby to achieve the functions of an image generation means 41, a region setting means 42, a temperature calculation means 43, a direction estimation means 44, and a rate estimation means 45 (illustrated in FIG. 3), which will be described later.

The storage device 34 may be, for example, a nonvolatile storage device, such as a flash memory, an SSD, a magnetic storage device (e.g., an HDD or the like), or an optical disk, or a volatile storage device, such as a RAM, and stores programs executed by the CPU 31 and data referred to by the CPU 31. The storage device 34 also stores a conversion table 50, which will be described later.

The display processing unit 35 displays data for display provided from the CPU 31 on the display unit 36. A liquid crystal display, for example, can be used as the display unit 36.

The input unit 37 is provided with a set of buttons for accepting the operation input from a user and includes an interface circuit for recognizing the pressing (operation) input of each button and outputting it to the CPU 31, but a touch panel input scheme may be adopted for the input unit 37.

The thermography camera 2 is connected to the communication interface unit 38, and the estimation device 3 is configured to be able to acquire a temperature distribution image transmitted from the thermography camera 2. Additionally or alternatively, the light source 1 may be connected to the communication interface unit 38, and the estimation device 3 may be configured to be able to control the irradiation of the subject's eye E with the near-infrared light from the light source 1 (ON/OFF of the light source 1), or another terminal device or the like may be connected to the communication interface unit 38, and the estimation device 3 may be configured to be able to transmit the estimation result of the fluid state of the aqueous humor to that terminal device or the like.

The functions achieved by the estimation device 3 configured as above will be described with reference to FIG. 3. FIG. 3 is a functional block diagram for describing functions that play major roles in the estimation device 3 according to the embodiment. The estimation device 3 may be composed of the image generation means 41, the region setting means 42, the temperature calculation means 43, the direction estimation means 44, and the rate estimation means 45. All of these means may not be essential components of the estimation device 3, and the functions of the estimation device 3 can be modified as appropriate in consideration of the type of liquid to be heated, the surrounding environment, weather conditions, the application of the system 100, the calculation load of the estimation device 3, etc.

The image generation means 41 has a function of generating a processing target image D_{P} used for estimating the fluid state of aqueous humor from among a plurality of temperature distribution images D acquired by the estimation device 3. This function is achieved, for example, as follows. It is assumed that the thermography camera 2 acquires the temperature distribution information of the subject's eye E at image capture intervals of 1 frame per second for 20 seconds from the time point at which the light source 1 starts the irradiation, and the estimation device 3 acquires a total of 20 temperature distribution images Dₙ (n=1 to 20).

First, the CPU 31 of the estimation device 3 selects one temperature distribution image to be a reference, for example, a temperature distribution image D₅ captured 5 seconds after the start of irradiation with the near-infrared light, as a reference image Dₛ from among the temperature distribution images Dₙ (n=1 to 20) of the subject's eye E acquired from the thermography camera 2 via the communication interface unit 38. The time to acquire the reference image Dₛ is not limited to 5 seconds after the start of heating and can be modified depending on the type of liquid to be heated, the heating method, the application of the system 100, etc. For example, the time to acquire the reference image Dₛ can be determined by experiments or the like. Additionally or alternatively, two or more temperature distribution images may be selected from among the acquired temperature distribution images Dₙ (n=1 to 20) of the subject's eye E, and an average image of the two or more temperature distribution images may be adopted as the reference image Dₛ.

Subsequently, the CPU 31 selects one temperature distribution image, for example, a temperature distribution image D₆ captured 6 seconds after the start of irradiation with the near-infrared light, from among the temperature distribution images Dₙ captured after the reference image Dₛ is captured, subtracts the reference image Dₛ from the temperature distribution image D₆ to generate a temperature distribution difference image, and adopts the temperature distribution difference image as a processing target image D_{P}. The number of the processing target image D_{P} to be generated is not limited to one, and two or more processing target images D_{P} may also be generated. For example, two processing target images D_{P} can be generated through selecting the temperature distribution image D₆ captured 6 seconds after the start of irradiation with the near-infrared light and a temperature distribution image D₇ captured 7 seconds after the start of irradiation with the near-infrared light and subtracting the reference image Dₛ from each of the temperature distribution image D₆ and the temperature distribution image D₇.

The processing target image D_{P} may not necessarily have to be the difference image generated from the reference image Dₛ and a temperature distribution image Dₙ, and the temperature distribution image Dₙ itself may be used as the processing target image D_{P} to estimate the fluid state of a liquid depending on the type of liquid to be heated, the heating method, the application of the system 100, etc. However, when estimating the fluid state of a liquid such as aqueous humor that flows very slowly and the rate at which the applied heat diffuses in the liquid is faster than the flow rate of the liquid itself, it may be advantageous to use the difference image generated from the reference image Dₛ and a temperature distribution image Dₙ as the processing target image D_{P}, and the fluid state can thereby be easily visualized.

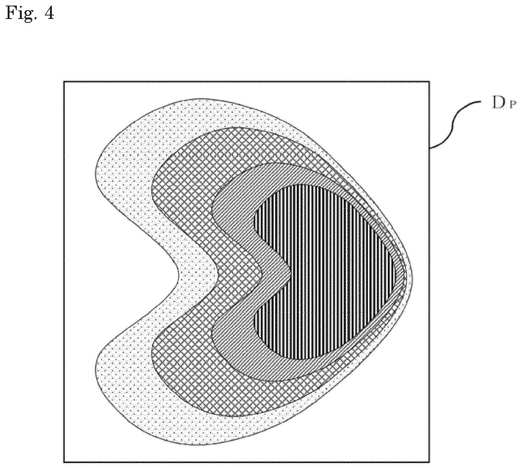

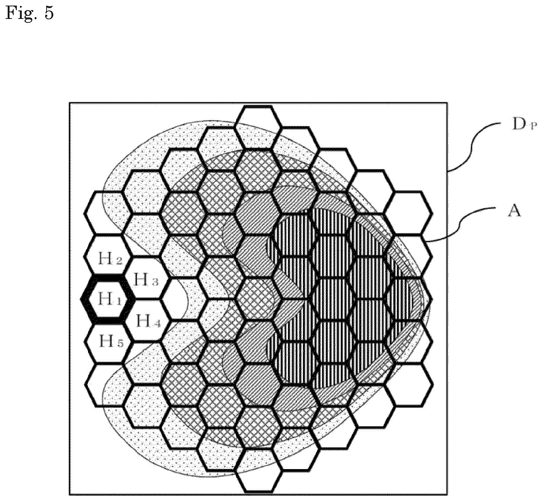

The region setting means 42 has a function of dividing the processing target image D_{P} into a plurality of unit regions, and this function is achieved, for example, as follows. When acquiring the processing target image D_{P} of the subject's eye E generated by the image generation means 41 as illustrated in FIG. 4, the CPU 31 of the estimation device 3 sets an analysis region A for the processing target image D_{P} as illustrated in FIG. 5.

The analysis region A is set with respect to the processing target image D_{P} so that, for example, the center position of the region coincides with the heating position by the light source 1 (position on the subject's eye E heated by the light source 1). In the present embodiment, as illustrated in FIG. 5, the analysis region A is formed by arranging regular hexagonal unit regions in a honeycomb shape so that the outer shape of the entire region is approximately a regular hexagon. By setting such an analysis region A for the processing target image D_{P}, the processing target image D_{P} can be divided into a plurality of unit regions.

The temperature calculation means 43 has a function of calculating the temperature of each unit region, and this function is achieved, for example, as follows. The CPU 31 of the estimation device 3 acquires, based on the processing target image D_{P}, the temperature of one unit region whose temperature is to be calculated and also acquires, based on the processing target image D_{P}, the temperature or temperatures of one or more other unit regions adjacent to the one unit region. Subsequently, the average value of these temperatures is calculated, and the calculated average value is adopted as the temperature of the one unit region. By calculating the temperature of the one unit region in consideration of the temperatures of the surrounding unit regions in such a way, it is possible to suppress the variation in temperature as compared to using the temperature of the one unit region alone, and the temperature of each unit region can therefore be calculated with a high degree of accuracy. The temperatures of all the adjacent unit regions may not necessarily have to be used in order to calculate the temperature of the one unit region, and the method of calculating the temperature can be modified as appropriate in consideration of the type of liquid to be heated, the surrounding environment, weather conditions, the application of the system 100, the calculation load of the estimation device 3, etc.

For example, assuming that a unit region H₁ illustrated in FIG. 5 is the target region for temperature calculation, the CPU 31 acquires a temperature T₁ of the unit region H₁ from the data of the processing target image D_{P} and also acquires temperatures T₂, T₃, T₄, and T₅ respectively of four unit regions H₂, H₃, H₄, and H₅ adjacent to the unit region H₁ from the data of the processing target image D_{P}. A temperature T_{A1} calculated by averaging all the five acquired temperatures (T₁, T₂, T₃, T₄, and T₅) is adopted as the temperature of the unit region H₁. The CPU 31 can calculate the temperatures of all the unit regions that constitute the analysis region A set in the processing target image D_{P} by using the same method.

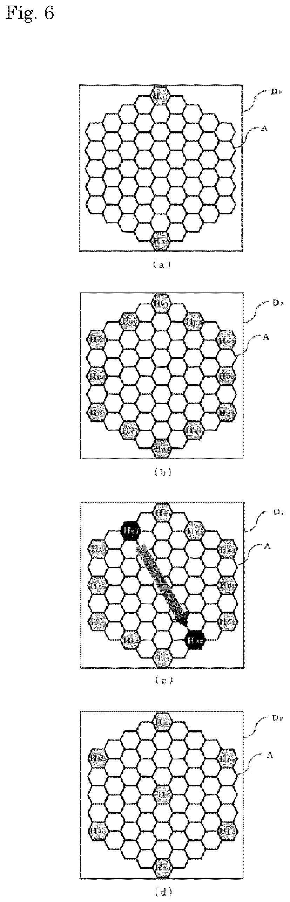

The direction estimation means 44 has a function of estimating the direction of an aqueous humor flow from the processing target image D_{P}, and this function is achieved, for example, as follows. First, the CPU 31 of the estimation device 3 sets two positions that form a pair within the analysis region A of the processing target image D_{P} and calculates the temperature difference between the two positions. For example, as illustrated in FIG. 6(a), two unit regions H_{A1} and H_{A2} located opposite to each other are set from among the unit regions located in the outer peripheral portion of the analysis region A, and the difference between the temperature of the unit region H_{A1} and the temperature of the unit region H_{A2} is calculated. The temperature distribution is not depicted in the processing target image D_{P} of FIG. 6 for the sake of easy understanding.

Likewise, as illustrated in FIG. 6(b), five pairs of two unit regions located opposite to each other are set, and the temperature difference between two unit regions that constitute each pair (unit regions H_{B1} and H_{B2}, H_{C1} and H_{C2}, H_{D1} and H_{D2}, H_{E1} and H_{E2}, H_{F1} and H_{F2} in FIG. 6(b)) is calculated. From among the six pairs of unit regions thus obtained, one pair having the maximum temperature difference between two unit regions is selected. Here, it is assumed that the temperature difference between the unit regions H_{B1} and H_{B2} is the maximum, and one pair composed of the two unit regions is selected.

Subsequently, the CPU 31 estimates, as the direction in which the aqueous humor flows, a direction from the unit region on the lower temperature side to the unit region on the higher temperature side of the two unit regions H_{B1} and H_{B2} constituting the selected one pair. For example, when the temperature of the unit region H_{B1} is lower than the temperature of the unit region H_{B2}, it is estimated that, as illustrated in FIG. 6(c), the aqueous humor flows in the direction from the unit region H_{B1} to the unit region H_{B2}. The estimated direction in which the aqueous humor flows is transmitted from the CPU 31 to the display processing unit 35 as the data for display and can be displayed using a directional image (e.g., an arrow) so that it is overlaid on the captured image of the subject's eye E displayed on the display unit 36 (including the temperature distribution image, the processing target image, the difference image, etc.).

In the present embodiment, two positions in each pair are set in the outer peripheral portion of the analysis region A so as to face each other (so as to be located on the diagonal line), but the present invention is not limited to this. For example, as illustrated in FIG. 6(d), one of the two positions forming each pair may be set at the center of the analysis region A with the other in the outer peripheral portion of the analysis region A, and the direction of the aqueous humor flow may be estimated based on the temperature difference between the center position of the analysis region A and each of a plurality of positions in the outer peripheral portion. In this example, six pairs each composed of a unit region H₀ at the center of the analysis region A and one of unit regions H₀₁, H₀₂, H₀₃, H₀₄, H₀₅, and H₀₆ in the outer peripheral portion are set, and the temperature difference between the two unit regions of each pair is calculated, such as a difference between the temperature of the unit region H₀ and the temperature of the unit region H₀₁ or a difference between the temperature of the unit region H₀ and the temperature of the unit region H₀₂.

Rather than using one unit region for each of the two positions to be paired, a set of unit regions formed of two or more unit regions may be used. In this case, the temperature of the set of unit regions may be the average temperature of the unit regions forming the set.

When the image generation means 41 has generated a plurality of processing target images D_{P}, the average value between the processing target images D_{P} regarding the temperature of each unit region obtained from the plurality of processing target images D_{P} may be adopted as the temperature of each unit region, and the direction of flow may be estimated as described above based on the average temperature. Alternatively, the direction of flow may be estimated as described above for each of the plurality of processing target images D_{P}, and the direction in which the aqueous humor flows may be estimated by comprehensively considering the obtained results.

The rate estimation means 45 has a function of estimating the rate at which the aqueous humor flows from the temperature difference between the two positions used by the direction estimation means 44 to estimate the direction in which the aqueous humor flows. This function is achieved, for example, as follows.

The CPU 31 of the estimation device 3 cross-checks, with the conversion table 50 stored in the storage device 34, the temperature difference between the two positions used by the direction estimation means 44 to estimate the direction in which the aqueous humor flows, that is, the temperature difference between the unit regions H_{B1} and H_{B2}, and estimates the flow rate derived from the temperature difference as the rate at which the aqueous humor flows. Specifically, the conversion table 50 is preliminarily created by quantifying the relationship between the amount of change in the temperature difference between two positions and the flow rate using a model for measuring the temperature difference between the two positions at various flow rates and defines the relationship between the temporal change amount (slope) of the temperature difference and the flow rate. By cross-checking, with the conversion table 50, the amount of change in the temperature difference between two positions actually acquired by the estimation device 3, the rate at which the aqueous humor flows can be estimated. The estimated rate at which the aqueous humor flows is transmitted from the CPU 31 to the display processing unit 35 as the data for display and can be displayed in the vicinity of the captured image of the subject's eye E and the directional image which are displayed on the display unit 36.

Table 1 represents an example of the conversion table 50. In the conversion table 50 of Table 1, the relationship between the amount of change in the temperature difference between two positions and the flow rate is quantified using a model for measuring the temperature difference between the two positions at flow rates of 0 µL/min (no flow), 5 µL/min, 10 µL/min, 15 µL/min, and 20 µL/min, and such a conversion table 50 is preliminarily created and stored in the storage device 34. Additionally or alternatively, regression analysis may be preliminarily performed based on the relationship between the amount of change in the temperature difference and the flow rate, and the obtained regression equation may be used for the conversion table 50.

**[Table 1]**

| Flow rate (µL/min) | Amount of change in temperature difference |
|---|---|
| 0 | 0 |
| 5 | 0.008 |
| 10 | 0.012 |
| 15 | 0.016 |
| 20 | 0.020 |

The CPU 31 obtains the amount of change in the temperature difference based on the temperature difference between the two positions used by the direction estimation means 44 to estimate the direction in which the aqueous humor flows, and estimates the flow rate corresponding to the amount of change in the temperature difference in the conversion table 50 as the rate at which the aqueous humor flows. For example, when the amount of change in the temperature difference based on the temperature difference between the two positions used by the direction estimation means 44 to estimate the direction in which the aqueous humor flows is calculated to be 0.0158, the closest value in the conversion table 50 is 0.016, and the CPU 31 therefore estimates the rate at which the aqueous humor flows to be 15 µL/min.

In the present embodiment, the estimation device 3 uses the direction estimation means 44 and the rate estimation means 45 to estimate the direction in which the aqueous humor flows and/or the rate at which the aqueous humor flows from the temperature difference between two positions in the analysis region A, but the temperature ratio may be used for estimation as substitute for the temperature difference.

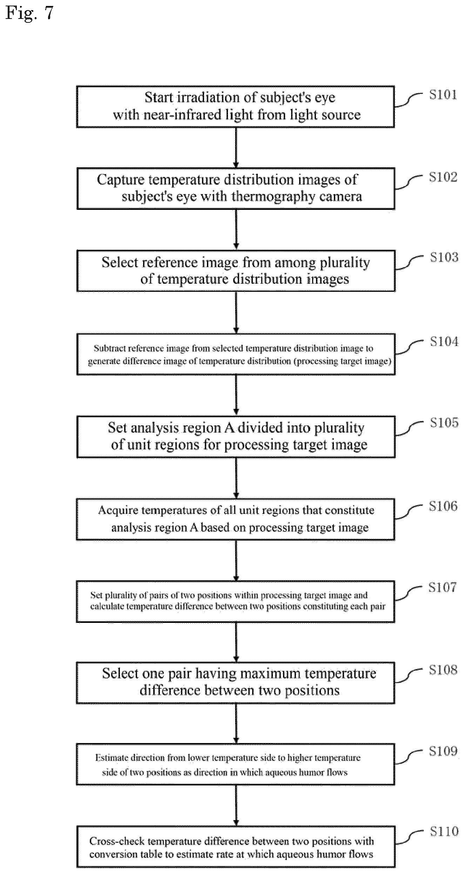

The flowchart illustrated in FIG. 7 will now be referred to for describing the main processing flow of the method for estimating the fluid state of a liquid which is performed by the system 100 configured as described above to estimate the fluid state of the aqueous humor of the subject's eye E.

First, the light source 1 is turned on to start irradiating the subject's eye E with near-infrared light (step S101). This allows the near-infrared light to pass through the cornea of the subject's eye E to enter the aqueous humor, and heating of the aqueous humor by the near-infrared light is started.

While continuing to irradiate the subject's eye E with near-infrared light from the light source 1, the thermography camera 2 captures images of the subject's eye E to acquire the temperature distribution information of the subject's eye E (its aqueous humor) (step S102). In the present embodiment, the thermography camera 2 acquires the temperature distribution information of the subject's eye E at image capture intervals of 1 frame per second for 20 seconds from the time point at which the light source 1 starts irradiation, and the temperature distribution information acquired by the thermography camera 2 is transmitted from the thermography camera 2 to the estimation device 3 as the temperature distribution images Dₙ (n=1 to 20) of the subject's eye E. The estimation device 3 acquires a total of 20 temperature distribution images of the subject's eye E and stores all or some of the temperature distribution images in the storage device 34 as necessary.

When acquiring the temperature distribution images Dₙ (n=1 to 20) of the subject's eye E from the thermography camera 2, the estimation device 3 selects the temperature distribution image D₅, which is captured 5 seconds after the start of irradiation with the near-infrared light, as the reference image Dₛ from among the temperature distribution images Dₙ (step S103). The estimation device 3 selects one temperature distribution image Dₙ from among the temperature distribution images Dₙ (n=1 to 20) other than the one adopted as the reference image Dₛ and subtracts the reference image Dₛ from the selected temperature distribution image Dₙ to generate a difference image of the temperature distribution, which is adopted as the processing target image D_{P} (step S104).

On the other hand, the estimation device 3 sets the analysis region A divided into a plurality of unit regions for the processing target image D_{P} (step S105). Subsequently, the estimation device 3 acquires, based on the processing target image D_{P}, the temperatures of all the unit regions that constitute the analysis region A (step S106). The temperature of one unit region in one processing target image D_{P} is calculated as an average value of the temperature of the one unit region obtained from the one processing target image D_{P} and the temperatures of a plurality of unit regions adjacent to the one unit region.

Subsequently, the estimation device 3 sets a plurality of pairs of two positions within the analysis region A of the processing target image D_{P} and calculates the temperature difference between the two positions constituting each pair (step S107). Then, the estimation device 3 selects, from among the plurality of pairs, one pair having the maximum temperature difference between the two positions constituting the pair (step S108) and estimates, as the direction in which the aqueous humor flows, a direction from the position on the lower temperature side to the position on the higher temperature side of the two positions constituting the selected one pair (step S109).

In addition, the estimation device 3 cross-checks, with the conversion table 50 stored in the storage device 34, the temperature difference between the two positions used to estimate the direction in which the aqueous humor flows, and estimates the flow rate derived from the temperature difference as the rate at which the aqueous humor flows (step S110).

Thus, the system 100 according to the present embodiment and the method for estimating the fluid state of a liquid carried out using the system 100 perceive the temperature distribution of the liquid, to which heat is applied, while taking advantage of the property that heat can be applied non-invasively to the liquid, and can thereby non-invasively estimate the fluid state of the aqueous humor, that is, the direction and flow rate of the aqueous humor flow.

In the above-described embodiment, the temperature distribution of the aqueous humor is acquired during the heating period in which heat is applied to the aqueous humor by the irradiation with near-infrared light from the light source 1, and the fluid state of the aqueous humor is estimated based on the temperature distribution during the heating period, but another embodiment is also possible in which the irradiation with near-infrared light from the light source 1 is stopped after the application of heat to the aqueous humor, the temperature distribution of the aqueous humor is acquired during a cooling period after the application of heat to the aqueous humor is stopped, and the fluid state of the aqueous humor is estimated based on the temperature distribution during the cooling period, or both the temperature distribution during the heating period and the temperature distribution during the cooling period are acquired and the fluid state of the aqueous humor is estimated based on both the temperature distributions.

Here, in the system 100 according to the present embodiment and the method for estimating the fluid state of a liquid carried out using the system 100, it is possible to estimate the fluid state of the liquid, for example, the presence or absence of a liquid flow and its flow direction, even from acquired one temperature distribution information item of the liquid (one temperature distribution image). On the other hand, when the fluid state of the liquid is comprehensively estimated based on a plurality of temperature distribution information items (a plurality of temperature distribution images acquired at different timings), the estimation accuracy can be improved as compared with the case in which the fluid state of the liquid is estimated from one temperature distribution information item.

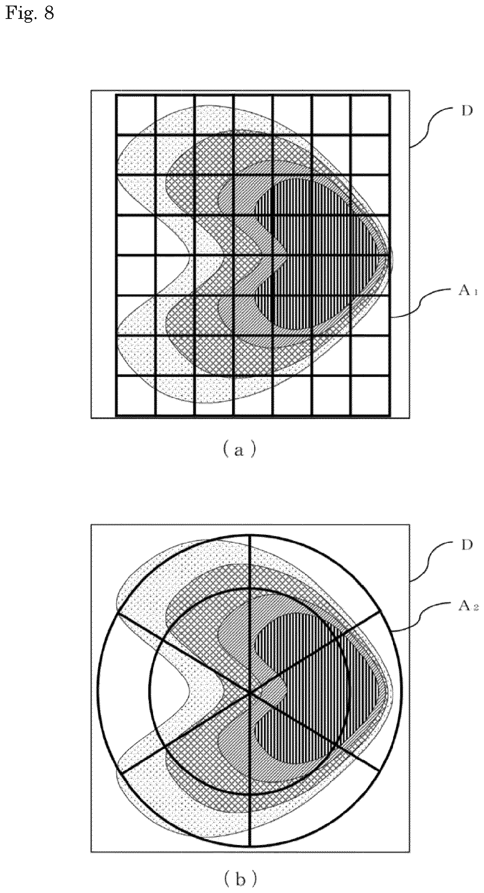

While the method for estimating the fluid state of a liquid and the system for estimating the fluid state of a liquid according to the present invention have been described above, the present invention is not limited to the above embodiment, and various modifications can be made and carried out. For example, in the above embodiment, the analysis region is set for the temperature distribution image by arranging a plurality of regular hexagonal unit regions in a honeycomb shape, but the present invention is not limited to this, provided that the direction of a liquid flow can be accurately estimated in the analysis region. For example, as illustrated in FIG. 8 (a), an analysis region A₁ composed of a plurality of square unit regions may be set, or as illustrated in FIG. 8 (b), a circular analysis region A₂ may be set by dividing concentric circles into a plurality of unit regions so that the area of the unit regions is the same. Additionally or alternatively, each pixel may be used as a unit region to set the analysis region.

In the above embodiment, the thermography camera 2 acquires the temperature distribution images of the subject's eye E at image capture intervals of 1 frame per second, but the present invention is not limited to this. For example, the temperature distribution images may be acquired at image capture intervals of 10 frames per second, or the average image of a plurality of contiguous frames (e.g., 3 frames) may be used as a temperature distribution image processed by the estimation device 3 to estimate the fluid state of aqueous humor. Thus, by using the average image of the temperature distribution images of several frames around a frame, it is possible to reduce the variation in the image for each image capture and the variation in the temperature over time.

The method for estimating the fluid state of a liquid and the system for estimating the fluid state of a liquid according to the present invention are used for estimating the fluid state of aqueous humor in the above embodiment, but the field of application is not limited to this. For example, other fields of application include perceiving the flow state of water or oil in order to find the presence or absence of water or oil leaks and the site of their causes, perceiving the flow state of blood in the human body, verification of the processing accuracy of microchannels, etc.

### [Examples]

The method for estimating the fluid state of a liquid and the system for estimating the fluid state of a liquid according to the present invention will be described in more detail below with reference to examples, but the scope of the present invention is not limited to these examples or the like.

### <Evaluation Device>

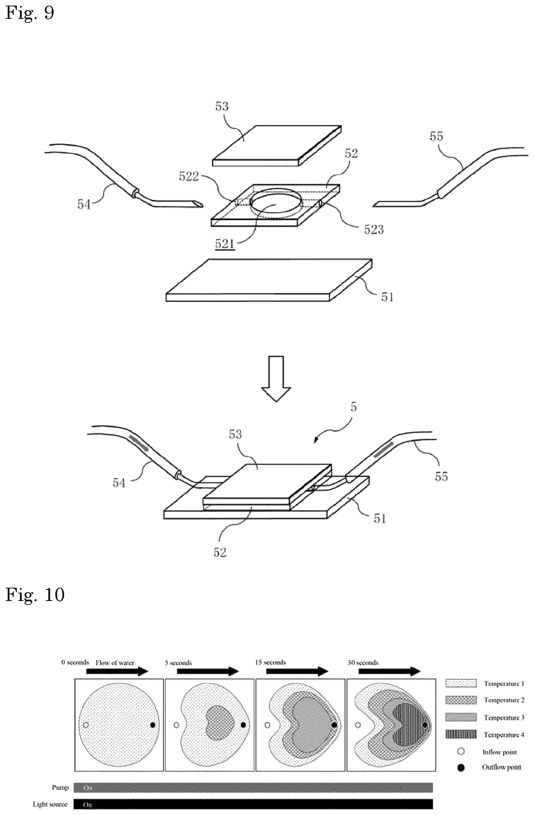

An evaluation device 5 as illustrated in FIG. 9 was used to conduct experiments to estimate the fluid state of a liquid (water). The evaluation device 5 includes a glass substrate 51, a resin gasket 52, and a cover glass 53 that are integrated. The gasket 52 is formed with a circular liquid flow space 521 in a plan view and is disposed on the glass substrate 51. The cover glass 53 is disposed on the gasket 52. One side portion (left side in FIG. 9) of the liquid flow space 521 of the gasket 52 is provided with an inflow port 522 into which an inflow pipe 54 for supplying the liquid to the gasket 52 is inserted, and the side portion (right side in FIG. 9) opposite to the one side portion of the liquid flow space 521 is provided with an outflow port 523 into which an outflow pipe 55 for discharging the liquid from the gasket 52 is inserted. The evaluation device 5 is configured to supply the liquid from the inflow port 522 of the gasket 52 using a pump (not illustrated, MINI-PUMP VARIABLE FLOW available from Fisher Scientific) and discharge the liquid from the outflow port 523, thereby allowing the liquid to flow in the liquid flow space 521 of the gasket 52. By controlling the pump, the flow rate of the liquid in the liquid flow space 521 can be controlled to a desired rate.

The evaluation device 5 is configured such that the center of the liquid flow space 521 of the gasket 52 is irradiated with near-infrared light from a near-infrared light source (not illustrated, an illumination device for infrared halogen lamp available from SUMITA OPTICAL GLASS, Inc.) and a thermography camera (not illustrated, Boson 320, 92° (HFOV) 2.3 mm available from FLIR Systems) can capture temperature distribution images of the liquid in the liquid flow space 521.

### <Example 1>

With the use of such an evaluation device 5, experiments were conducted to estimate the fluid state of water as a liquid whose fluid state was to be estimated. First, water was flowed in the liquid flow space 521 at a flow rate of 20 µL/min, and near-infrared light irradiation with an emission intensity of 24.6 mW was started to heat the water. During the heating, the thermography camera was used to capture the temperature distribution images of the water in the liquid flow space 521 at the time of start of irradiation (0 seconds) and 5 seconds, 15 seconds, and 30 seconds after the start of irradiation. FIG. 10 illustrates schematic representation of the four captured temperature distribution images. FIG. 10 illustrates how the temperature distribution of continuously heated water varies over time in a state in which the flow rate of water is fixed.

### <Example 2>

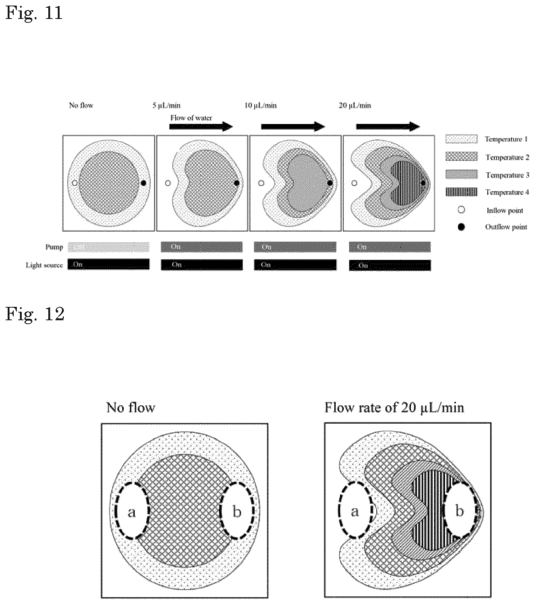

Then, using such an evaluation device 5, irradiation with near-infrared light was started to heat the water with four patterns in which the flow rate of water flowing in the liquid flow space 521 was changed from 0 µL/min (no flow) to 5 µL/min, 10 µL/min, and 20 µL/min, and the temperature distribution images of water in the liquid flow space 521 after 15 seconds from the start of irradiation were captured with the thermography camera. FIG. 11 illustrates schematic representation of the four captured temperature distribution images. FIG. 11 illustrates how the temperature distribution differs due to the difference in the flow rate of water at a time point at which a given time has elapsed from the start of heating.

From the temperature distribution images captured in Examples 1 and 2, it can be found that, by applying heat to the liquid and acquiring the temperature distributions of the liquid to which heat has been applied, the fluid state of the liquid can be estimated based on the acquired temperature distributions.

### <Quantification of Fluidity of Water>

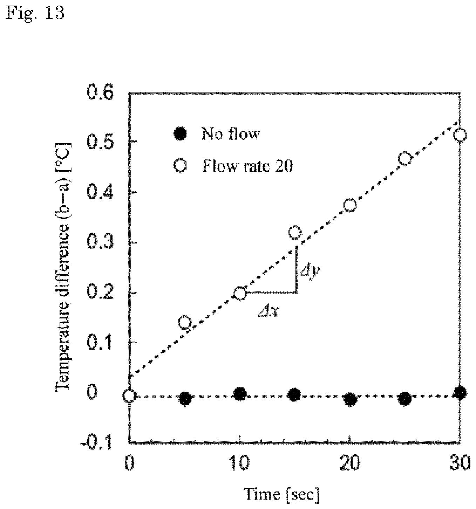

An attempt was made to quantify the fluidity of water from the temperature distribution image obtained with the pattern of each flow rate in Example 2. First, two regions "a" and "b" as illustrated in FIG. 12 were set for each of the temperature distribution images captured at image capture intervals of 5 seconds in the pattern with a flow rate of 0 µL/min (no flow) and the temperature distribution images captured at image capture intervals of 5 seconds in the pattern of a flow rate of 20 µL/min, and the temperatures of the regions "a" and "b" were acquired using the scheme of the above-described embodiment to calculate the temperature difference between the regions "a" and "b." When the temperature difference between the regions "a" and "b" at the time of start of heating water (0 seconds) and after 5 seconds, 10 seconds, 15 seconds, 20 seconds, 25 seconds, and 30 seconds elapsed from the start of heating was plotted on a graph with the temperature difference set on the vertical axis and the elapsed time set on the horizontal axis, the graph was able to be obtained as illustrated in FIG. 13. It can be understood from FIG. 13 that a state with no temperature difference between the regions "a" and "b" continues in the pattern of a flow rate of 0 µL/min (no flow) while the plots of the pattern of a flow rate of 20 µL/min are almost aligned on a straight line rising to the right.

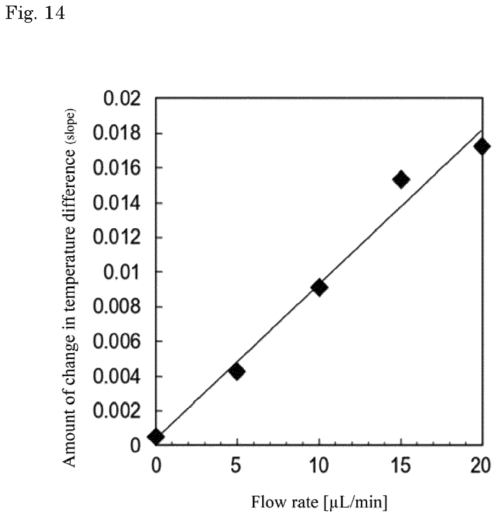

In addition to the four flow rate patterns of Example 2, the same experiments were conducted with a pattern in which the flow rate of water flowing in the liquid flow space 521 was set to 15 µL/min, and a similar graph to FIG. 13 was created for the total of five flow rate patterns to calculate a slope (Δy/Δx) of the temperature difference for each flow rate pattern. When the calculated slope (Δy/Δx) of the temperature difference for each flow rate pattern was plotted as an amount of change in the temperature difference for each flow rate pattern on a graph with the amount of change in the temperature difference set on the vertical axis and the flow rate set on the horizontal axis, the graph was able to be obtained as illustrated in FIG. 14. It can be understood from FIG. 14 that the amount of change in the temperature difference is determined in proportion to the flow rate, and it can be found that the conversion table 50 in the above-described embodiment can be preliminarily created by conducting such experiments.

### [Description of Reference Numerals]

- 100: System for estimating fluid state of liquid
- 1: Light source (heating means)
- 2: Thermography camera (temperature distribution acquisition means)
- 3: Estimation device (estimation means)
- 41: Image generation means
- 42: Region setting means
- 43: Temperature calculation means
- 44: Direction estimation means
- 45: Rate estimation means
- E: Subject's eye
- 5: Evaluation device

## Claims

1. A method for estimating a fluid state of a liquid, comprising:
a step (a) of applying heat to the liquid;
a step (b) of acquiring a temperature distribution of the liquid to which the heat is applied; and
a step (c) of estimating the fluid state of the liquid based on the acquired temperature distribution.

2. The method according to claim 1, wherein
the step (b) includes acquiring a plurality of temperature distributions of the liquid at different time points, and
the step (c) includes estimating the fluid state of the liquid based on the acquired plurality of temperature distributions.

3. The method according to claim 1 or 2, wherein the step (c) comprises:
a step (c1) of setting two positions to obtain a temperature difference or temperature ratio between the two positions, the two positions forming a pair within a region in which the temperature distribution is acquired;
a step (c2) of executing the step (c1) a plurality of times to select one pair from among a plurality of pairs of the two points within the region in which the temperature distribution is acquired, the one pair having a maximum temperature difference or temperature ratio; and
a step (c3) of estimating, as a direction in which the liquid flows, a direction from a position on a lower temperature side to a position on a higher temperature side of the two positions constituting the selected one pair.

4. The method according to claim 3, wherein the step (c) further comprises
a step (c4) of estimating a rate at which the liquid flows from the temperature difference or temperature ratio between the two positions constituting the one pair selected in the step (c2).

5. The method according to any one of claims 1 to 4, wherein the step (c) includes dividing a region in which the temperature distribution is acquired into a plurality of unit regions and estimating the fluid state of the liquid based on temperatures of the plurality of unit regions.

6. The method according to claim 5, wherein the temperature of one of the unit regions is calculated as an average value of the temperature of the one of the unit regions determined by the temperature distribution and the temperature of another unit region adjacent to the one of the unit regions.

7. The method according to any one of claims 1 to 6, wherein
the step (b) includes acquiring at least one of the temperature distribution of the liquid during a heating period in which the heat is applied to the liquid and the temperature distribution of the liquid during a cooling period after application of the heat to the liquid is stopped, and
the step (c) includes estimating the fluid state of the liquid based on at least one of the temperature distribution during the heating period and the temperature distribution during the cooling period.

8. The method according to any one of claims 1 to 7, wherein the step (a) includes irradiating the liquid with light from a light source thereby to apply the heat to the liquid.

9. The method according to any one of claims 1 to 8, wherein the step (b) includes capturing an image of the liquid with a thermography camera thereby to acquire the temperature distribution of the liquid.

10. A system for estimating a fluid state of a liquid, comprising:
a heating means that applies heat to the liquid;
a temperature distribution acquisition means that acquires a temperature distribution of the liquid to which the heat is applied; and
an estimation means that estimates the fluid state of the liquid based on the acquired temperature distribution.
